# EUROPEAN PATENT APPLICATION

(11) **EP 4 534 585 A1**
(43) Date of publication of application: **09.04.2025**
(21) Application number: 23815831.5
(22) Date of filing: 19.05.2023
(51) Int. Cl.: C08J 3/00, A61K 8/73, A61Q 1/12, C08B 15/08, C08L 1/02, C08L 83/04

(54) **CELLULOSE-SILOXANE COMPOSITE PARTICLES, METHOD FOR PRODUCING SAME, AND COSMETIC**

(30) Priority: 30.05.2022 JP 2022087689
(71) Applicant: Nissin Chemical Industry Co., Ltd., Fukui 915-0802 (JP); SHIN-ETSU CHEMICAL CO., LTD., Tokyo 1000005 (JP)
(72) Inventor: MITSUISHI Hirofumi, Echizen-shi, Fukui 915-0802 (JP); WATANABE Kentaro, Echizen-shi, Fukui 915-0802 (JP); INOKUCHI Yoshinori, Annaka-shi, Gunma 379-0224 (JP)
(74) Representative: De Vries & Metman
(86) International application number: PCT/JP2023/018750
(87) International publication number: WO 2023/234072

(57) **Abstract**

[Problem] To provide fine particles having excellent biodegradability and texture by coating biodegradable cellulose particles with silicone that imparts slidability, water repellency, and the like to form composite particles.

[Solution] The present invention provides: composite particles which are characterized by comprising particles of cellulose or a cellulose derivative and polyorganosilsesquioxane attached to the surfaces of the particles, wherein the poly- organosilsesquioxane is a polymer of organotrialkoxysilane in an amount of 3-80 parts by mass per 100 parts by mass of the particles; a method for producing the composite particles; and a cosmetic containing the composite particles.

## Description

### TECHNICAL FIELD

The present invention relates to cellulose-siloxane composite particles, a method for preparing the same, and cosmetics.

### BACKGROUND OF THE INVENTION

Conventionally, various polymeric fine particles have been proposed depending on the application. For example, the fine particles contained in cosmetics have various purposes. The purposes of having cosmetics contain fine particles are, for example, to improve the spread ability of the cosmetics, to impart changes to the tactile sensation, to impart a wrinkle-concealing effect, or to improve the lubricity of foundations.

In particular, fine particles with high sphericity have an excellent tactile sensation, and, depending on their physical properties and shape, they can provide a light scattering (soft focus) effect. Thus, when these fine particles are used in foundations or the like, they can be expected to have an effect that makes wrinkles and the like less noticeable (soft focus) by filling in and smoothing out any unevenness in the skin and by scattering light in various directions.

In order to achieve the purpose and effects of these cosmetics, the fine particles blended into cosmetics need to have a narrow particle size distribution and high sphericity. Fine particles composed of synthetic polymers such as polyamides like Nylon 12, polymethyl methacrylate (PMMA), and polystyrene (PS) have been proposed as such particles.

However, since fine particles composed of these synthetic polymers are lightweight with a relative density of 1 or less and have exceedingly small particle sizes, they easily float on water and may be unable to be removed at wastewater treatment plants. As a result, these fine particles may flow directly into rivers and further into oceans through said rivers. This poses a problem as oceans and other bodies of water are being polluted with fine particles composed of these synthetic polymers.

Further, fine particles composed of these synthetic polymers have properties in which they adsorb trace amounts of chemical pollutants in the environment, raising concerns that the fine particles may impart a variety of effects, including possible adverse effects on the human body caused by plankton and fish ingesting the fine particles that adsorbed these chemical pollutants.

Because of these concerns, attempts have been made to replace the fine particles composed of synthetic polymers used in a variety of applications with biodegradable particles.

Cellulose is a classic biodegradable resin. Cellulose is excellent in that it can be obtained from natural materials such as wood or cotton without conflicting with food or feed resources. Therefore, the development of fine particles that contain cellulose is desired.

Further, a synthetic resin powder made primarily from silicone is used in many cosmetics and the like, including hair cosmetics, makeup cosmetics, and sunscreens, as an important component that forms a uniform coating on the surface of skin and hair to moisturize and smooth the skin and hair, as well as to impart water repellency and water resistance to the skin and hair. For example, Japanese Patent Application Laid-Open No. Hei 07-196815 (Patent Literature 1) discloses fine silicone particles that have a soft tactile sensation and excellent dispersibility without any aggregation. Although the silicone fine particles are suitably blended into cosmetics, there is still a demand for the development of a better resin powder.

WO2020/188698 discloses that a cellulose acetate particle is surface-treated with a lipophilicity-imparting agent including a silicone-based component and then blended into a cosmetic composition. However, this method attaches the lipophilicity-imparting agent to the cellulose acetate particle using a wet processing method and requires the use of an organic solvent such as n-hexane. Thus, there is a demand for the development of a composite particle under more environmentally friendly aqueous conditions.

### PRIOR LITERATURES

### PATENT LITERATURES

Patent Literature 1: Japanese Patent Application Laid-Open No. Hei 07-196815 (1995)
Patent Literature 2: WO2020/188698

### DISCLOSURE OF THE INVENTION

### PROBLEMS TO BE SOLVED BY THE INVENTION

The present invention has been made in consideration of the aforesaid circumstances, and an object of the present invention is to provide a fine particle that is excellent in terms of biodegradability and tactile sensation by coating a biodegradable cellulose particle with silicone that imparts slide ability, water repellency, and the like, thereby forming a composite particle.

### SOLUTIONS TO THE PROBLEMS

As a result of extensive studies for achieving the aforesaid object, the present inventors have developed a composite particle in which a cellulose particle is coated with a polyorganosilsesquioxane, which led to the present invention.

That is, the present invention provides a composite particle composed of a particle of cellulose or a cellulose derivative and a polyorganosilsesquioxane attached to the surface of the particle, wherein the polyorganosilsesquioxane is a polymer of 3 to 80 parts by mass of organotrialkoxysilane, relative to 100 parts by mass of the particle, a method for preparing the composite particle, and a cosmetic including the composite particle.

### EFFECTS OF THE INVENTION

When the composite particle of the present invention is blended into and used in a cosmetic, the cosmetic is imparted with slide ability, a soft tactile sensation, water repellency, and the like. Thus, the composite particle of the present invention is suitable to blend into many cosmetics such as hair cosmetics, makeup cosmetics, and sunscreens.

### BRIEF DESCRIPTION OF THE DRAWINGS

FIG. 1 is an electron microscopy image of the surface of a composite particle obtained in Example 4.
FIG. 2 shows an enlarged electron microscopy image (a) of the surface of the composite particle obtained in Example 4, and an image (b) in which an elemental mapping image of silicon and an elemental mapping image of carbon are superimposed.
FIG. 3 is an electron microscopy image of the surface of a composite particle obtained in Comparative Example 5.

### DETAILED DESCRIPTION OF THE INVENTION

The present invention provides a composite particle composed of a particle of cellulose or a cellulose derivative and a polyorganosilsesquioxane attached to the surface of the particle, wherein the polyorganosilsesquioxane is a polymer of 3 to 80 parts by mass of organotrialkoxysilane, preferably 5 to 70 parts by mass, or more preferably 15 to 55 parts by mass of organotrialkoxysilane, relative to 100 parts by mass of the particle.

The details are described below.

### (A) Particle of cellulose or a cellulose derivative

A particle of cellulose or a cellulose derivative (hereinafter collectively referred to as a cellulose particle) is a particle that includes cellulose. The particle may be a particle of a cellulose derivative such as cellulose acetate or cellulose acetate propionate. Further, the shape of the cellulose particle is not limited to a spherical shape, and the cellulose particle is conventionally spherical. In the present invention, a spherical cellulose particle is preferable.

The volume average particle size of the cellulose particle is preferably 1 to 300 µm. Further, the volume average particle size is more preferably 1 to 150 µm or still more preferably 1 to 50 µm. More specifically, examples of the volume average particle size include sizes of 3 µm, 5 µm, 7 µm, 10 µm, 15 µm, 20 µm, 45 µm, and the like. The volume average particle size of the cellulose particle refers to a particle size at D50% of integrated volume determined from data obtained by measurements using a laser diffraction particle size distribution analyzer, e.g., "SALD2100", provided by SHIMADZU CORPORATION.

The cellulose particle or the cellulose derivative is produced, for example, by suspending a cellulose acetate solution, in which cellulose acetate is dissolved in an organic solvent, in water to prepare a suspension in which cellulose acetate particles are dispersed in water, removing the organic solvent from the suspension, and saponifying the cellulose acetate particles such that the amount of acetic acid is 0.5 ppm or less, relative to the mass of the cellulose. After the cellulose acetate particles are saponified to produce cellulose particles, water may be further removed by solid-liquid separation to dry the cellulose particles.

Examples of a commercially available cellulose particle include BELLOCEA, trademark, cellulose acetate provided by Daicel Corporation; Viscopearl, trademark, provided by Rengo Co., Ltd.; and ART PEARL NC-400 and NC-800, provided by Negami Chemical Industrial Co., Ltd.

### (B) Organotrialkoxysilane

The composite particle of the present invention is formed by attaching a polyorganosilsesquioxane to the surface of the aforesaid cellulose particle. The polyorganosilsesquioxane is a polymer of an organotrialkoxysilane. The organotrialkoxysilane is represented by the following formula.

R¹Si(OR²)₃

In the formula, R¹ is an alkyl group having 1 to 30 carbon atoms, and R² is a hydrogen atom or an alkyl group having 1 to 4 carbon atoms. The alkyl group is most preferably a methyl group. In the formula, R¹ is an alkyl group having 1 to 30 carbon atoms, preferably 1 to 12 carbon atoms, or more preferably 1 to 8 carbon atoms. The alkyl group is most preferably a methyl group. The organotrialkoxysilane may be used alone or in combination of two or more kinds.

The aforesaid organotrialkoxysilane is preferably a methyl trimethoxysilane, methyl triethoxysilane, methyl tripropoxysilane, ethyl trimethoxysilane, ethyl triethoxysilane, ethyl tripropoxysilane, ethyl tributoxysilane, propyl trimethoxysilane, propyl triethoxysilane, isobutyl trimethoxysilane, isobutyl triethoxysilane, butyl trimethoxysilane, butyl triethoxysilane, hexyl trimethoxysilane, n-octyl triethoxysilane, n-octyl trimethoxysilane, i-octyl trimethoxysilane, or i-octyl triethoxysilane.

The organotrialkoxysilane may be well known and commercially available. For example, such organotrialkoxysilanes are described in U.S. Patent No. 5,300,327 (April 5, 1994), U.S. Patent No. 5,695,551 (December 9, 1997), and U.S. Patent No. 5,919,296 (July 6, 1999).

The organotrialkoxysilane is particularly preferably a methyl trimethoxysilane in which R¹ and R² are methyl groups.

### Preparation method

The present invention provides a method for preparing a composite particle composed of a particle of cellulose or a cellulose derivative (A) and a polyorganosilsesquioxane attached to the surface of the particle, wherein the method comprising a step of subjecting 3 to 80 parts by mass of an organotrialkoxysilane (B) relative to 100 parts by mass of the particle (A) to a hydrolysis and condensation reaction in the presence of the particle (A), water and an alkali, to form the polyorganosilsesquioxane, and then attach the polyorganosilsesquioxane thus obtained to the surface of the particle (A). The method includes a subsequent step of removing water. Here, the polyorganosilsesquioxane is a polymer composed of RSiO_{3/2} units. The preparation method of the present invention is described in detail below.

In step (i) of the preparation method of the present invention, the polyorganosilsesquioxane is formed by subjecting organotrialkoxysilane (B) to a hydrolysis and condensation reaction in the presence of cellulose particle (A), water, and the alkali. The polyorganosilsesquioxane obtained by the hydrolysis and condensation reaction is in a state in which the polyorganosilsesquioxane is attached to the cellulose particle.

The alkali acts as a catalyst for the hydrolysis and condensation reaction of or as a catalyst for the condensation reaction of organotrialkoxysilane (B). The alkali may be used alone or in combination of two or more kinds. Also, the alkali may be added as is or as an aqueous alkaline solution. Further, the alkali may be blended into the aqueous dispersion including the cellulose particle and water before the organotrialkoxysilane is added, or the alkali may be added after the organotrialkoxysilane is added.

The amount of alkali added is such that the pH of the aqueous dispersion including the cellulose particle and water is within a range of preferably 9.0 to 13.0 or more preferably 9.5 to 12.5. When the pH is within the aforesaid range, the hydrolysis and condensation reaction of the organotrialkoxysilane sufficiently progresses, and the resulting polyorganosilsesquioxane sufficiently attaches to the surface of the cellulose particle.

The alkali is not particularly limited and is only required to progress the hydrolysis and condensation reaction of organotrialkoxysilane (B). Examples of the alkali include alkali metal hydroxides such as potassium hydroxide, sodium hydroxide, or lithium hydroxide; alkaline earth metal hydroxides such as calcium hydroxide or barium hydroxide; alkali metal carbonates such as potassium carbonate or sodium carbonate; ammonia; tetraalkyl ammonium hydroxides such as tetramethyl ammonium hydroxide or tetraethyl ammonium hydroxide; and amines such as monomethyl amine, dimethyl amine, diethyl amine, trimethyl amine, triethanol amine, or ethylene diamine. Among these, the most suitable alkali is ammonia because ammonia is easily removed from the resulting cellulose particle powder by evaporation. For the ammonia, a commercially available aqueous ammonia solution may be used.

The amount of organotrialkoxysilane (B) is an amount such that the amount of the polyorganosilsesquioxane is within a range of 3 to 80 parts by mass, preferably 5 to 70 parts by mass, or more preferably 15 to 55 parts by mass, relative to 100 parts by mass of the cellulose particle (A). If the amount of organotrialkoxysilane (B) is less than 1 part by mass, the effects of siloxane are not exhibited, whereas an amount exceeding the aforesaid upper limit generates an aggregated particle or a siloxane that does not cover the cellulose particle. An amount exceeding the aforesaid upper limit generates an aggregated particle or a siloxane that does not cover the cellulose particle, resulting in a worsened tactile sensation and a hard or granular sensation even when the particles are blended into a cosmetic.

Preferred is that the addition of organotrialkoxysilane (B) is conducted with stirring using a conventional stirrer such as a propeller or flat-blade stirrer.

Further, for the purpose of controlling the attachment of the polyorganosilsesquioxane to the surface of the cellulose particle, a surfactant or a water-soluble polymer may be added to the aqueous cellulose dispersion.

The surfactant added to the aqueous dispersion is not particularly limited, and examples thereof include a nonionic surfactant, anionic surfactant, cationic surfactant, and amphoteric surfactant. Two or more kinds of surfactant may be added. When the surfactant is added, the amount thereof is preferably within a range of 0.01 to 10 parts by mass, relative to 100 parts by mass of cellulose particle (A).

Examples of the nonionic surfactant include a polyoxyethylene alkyl ether, polyoxyethylene-polyoxypropylene alkyl ether, polyoxyethylene alkyl phenyl ether, polyethylene glycol fatty acid ester, sorbitan fatty acid ester, polyoxyethylene sorbitan fatty acid ester, polyoxyethylene sorbitol fatty acid ester, glycerol fatty acid ester, polyoxyethylene glycerol fatty acid ester, polyglycerol fatty acid ester, propylene glycol fatty acid ester, polyoxyethylene castor oil, polyoxyethylene hydrogenated castor oil, polyoxyethylene fatty acid amide, polyoxyethylene-modified organopolysiloxane, and polyoxyethylene-polyoxypropylene-modified organopolysiloxane.

Examples of the anionic surfactant include an alkyl sulfate ester salt, polyoxyethylene alkyl ether sulfate ester salt, polyoxyethylene alkyl phenyl ether sulfate ester salt, sulfate ester salt of fatty acid alkylol amide, alkyl benzene sulfonate salt, α-sulfofatty acid ester salt, alkyl naphthalene sulfonate, alkyl diphenyl ether disulfosuccinate salt, fatty acid salt, polyoxyethylene alkyl ether carboxylate salt, N-acyl amino acid salt, monoalkyl phosphate ester salt, dialkyl phosphate ester salt, and polyoxyethylene alkyl ether phosphate ester salt.

Examples of the cationic surfactant include an alkyl trimethyl ammonium salt, dialkyl dimethyl ammonium salt, polyoxyethylene alkyl dimethyl ammonium salt, dipolyoxyethylene alkyl methyl ammonium salt, tripolyoxyethylene alkyl ammonium salt, alkyl benzyl dimethyl ammonium salt, alkyl pyridinium salt, monoalkyl amine salt, and monoalkyl amidoamine salt.

Examples of the amphoteric surfactant include alkyl dimethyl amine oxide, alkyl dimethyl carboxybetaine, alkyl amidopropyl dimethyl carboxybetaine, alkyl hydroxysulfobetaine, and alkyl carboxymethyl hydroxyethyl imidazolinium betaine.

The water-soluble polymer added to the aqueous dispersion is not particularly limited, and examples thereof include a nonionic water-soluble polymer, anionic water-soluble polymer, cationic water-soluble polymer, and amphoteric water-soluble polymer. The water-soluble polymer may be used alone or in combination of two or more kinds. The amount of the water-soluble polymer is preferably within a range of 0.01 to 10 parts by mass, relative to 100 parts by mass of cellulose particle (A).

Examples of the nonionic water-soluble polymer include a copolymer of vinyl alcohol and vinyl acetate, acryl amide polymer, vinyl pyrrolidone polymer, copolymer of vinyl pyrrolidone and vinyl acetate, polyethylene glycol, isopropyl acryl amide polymer, methyl vinyl ether polymer, starch, methyl cellulose, hydroxyethyl cellulose, hydroxypropyl cellulose, guar gum, and xanthan gum.

Examples of the anionic water-soluble polymer include a dimethyl diallyl ammonium chloride polymer, vinyl imidazoline polymer, methyl vinyl imidazolium chloride polymer, trimethyl ammonium ethyl acrylate chloride polymer, trimethyl ammonium ethyl methacrylate chloride polymer, acryl amidopropyl trimethyl ammonium chloride polymer, methacryl amidopropyl trimethyl ammonium chloride polymer, epichlorohydrin/dimethyl amine polymer, ethylene imine polymer, quaternary ethylene imine polymer, allyl amine hydrochloride polymer, polylysine, cationic starch, cationic cellulose, chitosan, and derivatives thereof obtained by copolymerizing these with monomers having nonionic groups or anionic groups.

Examples of the amphoteric water-soluble polymer include a copolymer of trimethyl ammonium ethyl acrylate chloride, acrylic acid, and acryl amide, copolymer of trimethyl ammonium ethyl methacrylate chloride, acrylic acid, and acryl amide, and Hofmann degradation product of an acryl amide polymer.

In one embodiment of the preparation method of the present invention, the alkali is blended into the aqueous dispersion including cellulose particle (A) and water, and then organotrialkoxysilane (B) is added to the mixture. In this method, the organotrialkoxysilane may be added all at once, but preferred is that the organotrialkoxysilane is added gradually over a period of time. The reaction temperature when the organotrialkoxysilane is added is preferably within a range of 0°C to 60°C or more preferably 0°C to 40°C. If the temperature is within the aforesaid range, the polyorganosilsesquioxane successfully attaches to the surface of the cellulose particle. Stirring continues after the organotrialkoxysilane is added until the hydrolysis and condensation reaction of the organotrialkoxysilane are completed. In order to complete the hydrolysis and condensation reaction, the reactions may be performed at room temperature or under heating at about 40°C to 100°C. Further, the alkali may be added as appropriate.

In another embodiment of the preparation method of the present invention, the organotrialkoxysilane may be added before the alkali is added. In this method, preferred is that the organotrialkoxysilane is added to water at first. The organotrialkoxysilane may be added to the water all at once, or the organotrialkoxysilane may be added gradually to the water over a period of time. Alternatively, water may be added to the organotrialkoxysilane, or water and the organotrialkoxysilane may be placed in a tank at the same time and mixed. The temperature when the organotrialkoxysilane is added to the water is not particularly limited, and the temperature may be, for example, within a range of 0°C to 100°C. Subsequently, stirring continues until the hydrolysis reaction of the organotrialkoxysilane progresses and at least the organotrialkoxysilane is dissolved in the water. During this process, a small amount of acid may be added to promote the hydrolysis reaction.

Subsequently, an aqueous dispersion including the cellulose particle is added to the aforesaid mixed solution obtained, and then the alkali is added. The alkali added promotes the condensation reaction of the hydrolysate of the organotrialkoxysilane, to thereby obtain the polyorganosilsesquioxane. In this process, it is necessary that stirring is stopped or made very slowly before the polyorganosilsesquioxane is produced. If the reaction solution is stirred at a high speed during the preparation of the polyorganosilsesquioxane, the polyorganosilsesquioxane does not successfully attach to the cellulose particle.

The temperature during the condensation reaction is preferably within a range of 0°C to 60°C, more preferably 0°C to 40°C. When the temperature is within the aforesaid range, the polyorganosilsesquioxane successfully attaches to the cellulose particle. Preferred is that the reaction solution is left to rest or the reaction solution is stirred very slowly until the time when the polyorganosilsesquioxane is produced, that is, when the polyorganosilsesquioxane is attached to the surface of the cellulose particle. The reaction solution is left to rest for a period within a range of preferably 10 minutes to 24 hours. Subsequently, in order to complete the condensation reaction, the alkali may be further added, or the reaction mixture may be heated at a temperature within a range of 40°C to 100°C. Also, conventional stirring may be further performed.

In step (ii) of the preparation method of the present invention, the water is removed by evaporation after the polyorganosilsesquioxane attached to the surface of the cellulose particle is obtained.

The water may be removed via evaporation by heating under normal pressure or reduced pressure. Examples of such a method include a method in which the dispersion is left to rest under heating to remove the water. As a pre-treatment for this operation, the dispersion may be concentrated with a method such as filtration separation, centrifugation, or decantation, and if necessary, the dispersion may be washed with water, water-soluble alcohol, or the like.

When the powder of composite particles obtained by removing water via evaporation aggregates, the aggregated powder is crushed using a pulverizer such as a jet mill, ball mill, or hammer mill.

In the composite particle obtained by the aforesaid method, the cellulose particle is coated with the polyorganosilsesquioxane. Whether the cellulose particle is coated with the polyorganosilsesquioxane is confirmed with an infrared absorption spectrum. Further, the coating state is confirmed by observing the surface using electron microscopy images. In particular, preferred is that 30% or more of, more preferably 30% to 95% of, still more preferably 40% to 90% of, or even more preferably 40% to 80% of the surface area of the cellulose particle is covered with the polyorganosilsesquioxane. If the coverage rate of the surface of the cellulose particle is less than 30%, the effects of siloxane are not exhibited. If the coverage rate is too high and an aggregated particle or a siloxane that does not coat the cellulose particle generates, resulting in a worsened tactile sensation and a loss of the biodegradable effect of the cellulose particle. The volume average particle size of the composite particle is preferably 1 to 300 µm. Further, preferred is 1 to 150 µm, or still more preferably 1 to 50 µm.

The static and dynamic friction coefficients of a composite body composed of the composite particle are preferably 0.4 or less, and such a composite has good smoothness and lubricity. The composite particle blended into and used in a cosmetic described below provides the same effects to the cosmetic. The term "a composite body composed of the composite particle" refers to a product formed by, for example, uniformly spreading composite particles onto a substrate such as a BIOSKIN plate.

The composite particle of the present invention is blended into and used in a cosmetic, and the amount of the composite particle blended into the cosmetic is 1% to 50% by mass, preferably 1% to 30% by mass, relative to the entire cosmetic. If the amount is less than the lower limit value, the effects may not be able to be sufficiently obtained, whereas an amount exceeding the upper limit value may not be preferable due to undesirable results such as noticeable whiteness.

Examples of cosmetic components other than the composite particle of the present invention include an oil, solvent, and powder other than a silicone-based copolymer resin powder.

Examples of the oil include a hydrocarbon, silicone oil, triglyceride, ester oil, fats and oils, waxes, a higher fatty acid having 12 to 20 carbon atoms, and a higher alcohol having 8 to 20 carbon atoms. In particular, a silicone oil with a low boiling point, isoparaffin-based hydrocarbon with a low boiling point, triglyceride, and ester oil are preferable. Examples of the silicone oil with a low boiling point include octamethyl cyclotetrasiloxane, decamethyl cyclopentasiloxane, and tetradecamethyl cyclohexasiloxane. Examples of the ester oil include a fatty acid ester having 6 to 20 carbon atoms and a glycerol fatty acid ester.

The amount of the oil contained in the cosmetic varies depending on the dosage form of the cosmetic, and may be in a range as long as that the effects of the present invention are not impaired. The amount is preferably 0.1% to 95% by mass or more preferably 1% to 80% by mass, relative to the total amount of the powder and the resin powder component of the present invention. If the amount is less than the lower limit value, the lubricity, moisturizing properties, and the like of the oil may not be able to be exhibited, whereas an amount exceeding the upper limit value has a tendency for the storage stability to decrease.

Examples of the solvent include lower or intermediate alcohols and aromatic alcohols, and a lower alcohol having 1 to 4 carbon atoms, such as isopropyl alcohol, is preferable. The amount of the solvent contained in the cosmetic of the present invention varies depending on the dosage form of the cosmetic, and may be in a range as long as that the effects of the present invention are not impaired. The amount is preferably 0.1% to 80% by mass or more preferably 1% to 50% by mass, relative to the total amount of the powder and the resin powder component of the present invention.

The powder refers to a material used in conventional (makeup) cosmetics, and the powder is not particularly limited. Conventionally, the average particle size of the powder is 0.1 to 50 µm, and examples thereof include colorants such as an inorganic colored pigment, inorganic white pigment, or organic pigment; pearl pigment; extender pigment; and organic powder.

Examples of the powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, silicic acid, anhydrous silicic acid, aluminum silicate, sodium silicate, sodium magnesium silicate, magnesium silicate, magnesium aluminum silicate, calcium silicate, barium silicate, strontium silicate, a metal salt of tungstic acid, hydroxyapatite, vermiculite, higilite, bentonite, montmorillonite, hectorite, zeolite, a ceramic powder, dicalcium phosphate, alumina, aluminum hydroxide, and boron nitride. Examples of the organic powder include a polyamide powder, polyester powder, polyethylene powder, polypropylene powder, polystyrene powder, polyurethane powder, benzoguanamine powder, polymethyl benzoguanamine powder, polytetrafluoroethylene powder, polymethyl methacrylate powder, cellulose, silk powder, nylon powder, Nylon 12, Nylon 6, silicone powder, spherical polymethyl silsesquioxane powder, copolymer of styrene and acrylic acid, copolymer of divinyl benzene and styrene, vinyl resin, urea-formaldehyde resin, phenol-formaldehyde resin, fluorine resin, silicone resin, acrylic resin, melamine-formaldehyde resin, epoxy resin, polycarbonate resin, microcrystalline fiber powder, starch powder, and lauroyl lysine. Examples of the surfactant metal salt powder include zinc stearate, aluminum stearate, calcium stearate, magnesium stearate, zinc myristate, magnesium myristate, zinc palmitate, zinc laurate, zinc cetyl phosphate, calcium cetyl phosphate, and sodium zinc cetyl phosphate. Examples of the colored pigment include inorganic red pigments such as red iron oxide, iron oxide, iron hydroxide, or iron titanate; inorganic brown pigments such as γ-iron oxide; inorganic yellow pigments such as yellow iron oxide or ocher; inorganic black pigments such as black iron oxide or carbon black; inorganic purple pigments such as manganese violet or cobalt violet; inorganic green pigments such as chromium hydroxide, chromium oxide, cobalt oxide, or cobalt titanate; inorganic blue pigments such as navy blue or ultramarine blue; tar-based lake pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, or Orange No. 207; and natural lake pigments such as carminic acid, laccaic acid, carthamin, brazilin, or crocin. Examples of the pearl pigment include mica coated with titanium oxide, titanated mica, titanated mica treated with iron oxide, bismuth oxychloride, bismuth oxychloride coated with titanium oxide, talc coated with titanium oxide, fish scale flakes, and colored mica coated with titanium oxide. Examples of the metal powder pigment include powders of metals such as aluminum, gold, silver, copper, platinum, or stainless steel.

Further, in addition to the constituent components, other components that are blended into conventional cosmetics, such as a surfactant, oily component, polymer compound, gelling agent, alkaline agent, polyhydric alcohol, pH adjuster, ultraviolet absorber, antioxidant, preservative, anti-inflammatory agent, skin care component, and fragrance, may be blended into the cosmetic of the present invention, within quantitative and qualitative ranges that do not impair the effects of the composite particle of the present invention, depending on the purpose.

Examples of the cosmetic of the present invention include makeup cosmetics such as a foundation, face powder, eye shadow, eyeliner, eyebrow makeup, blush, lipstick, or nail polish color; foundation cosmetics such as a milky lotion, cream, lotion, calamine lotion, sunscreen, tanning lotion, aftershave lotion, pre-shave lotion, facial mask, anti-acne cosmetic, or essence; hair cosmetics such as a shampoo, rinse, conditioner, hair dye, hair tonic, hair styling product, hair care product, or perming product; a body powder; a deodorant; a hair removal product; a soap; a body shampoo; a bath salt or bath bomb; a hand soap; and a perfume. The silicone-based copolymer resin powder of the present invention is preferably used in powder cosmetics such as a foundation, face powder, eye shadow, or eyebrow makeup.

### EXAMPLES

The present invention will be explained below in further detail with reference to a series of the Examples and the Comparative Examples, though the present invention is in no way limited by these Examples. Hereinafter, "part" or "%" represents part by mass or mass%, respectively.

### [Example 1]

In a 500-ml reaction chamber, were placed 100 parts by mass of a spherical cellulose particle (A-1) having an average particle size of 7 µm, 830 parts by mass of deionized water, and 1.6 parts by mass of dodecyl trimethyl ammonium chloride (CATION BB, cationic emulsifier provided by NOF CORPORATION) and stirred for 30 minutes. Then, 22 parts by mass of 25% ammonia water were added to the mixture to adjust the pH to a value of 11 to 12 (measured using an HM-42X, a benchtop pH meter provided by DKK-TOA CORPORATION). After the resulting mixture was cooled to 5°C, 22.5 parts by mass of methyl trimethoxysilane (KBM13 provided by Shin-Etsu Chemical Co., Ltd.) was added to the mixture over a period of 20 minutes. After the mixture was incubated at 8°C for 1 hour, the temperature was raised to 55°C, 53 parts by mass of 25% ammonia water were added to the mixture, and the resulting mixture was incubated for 1 hour to obtain a slurry. The slurry was vacuum filtered to separate the solid and liquid contents, and then left to dry, resulting in a monodisperse, water-repellent powder. An electron microscopy confirmed that the powder obtained included a composite particle in which a polymethyl silsesquioxane was attached to the surface of a granular cellulose particle.

### [Example 2]

The procedures of Example 1 were repeated, except that the amount of methyl trimethoxysilane used was 15 parts by mass, to obtain a monodisperse, water-repellent powder. An electron microscopy confirmed that the powder obtained included a composite particle in which a polymethyl silsesquioxane was attached to the surface of a granular cellulose particle.

### [Example 3]

The procedures of Example 1 were repeated, except that the amount of methyl trimethoxysilane used was 55 parts by mass, to obtain a monodisperse, water-repellent powder. An electron microscopy confirmed that the powder obtained included a composite particle in which a polymethyl silsesquioxane was attached to the surface of a granular cellulose particle.

### [Example 4]

The procedures of Example 1 were repeated, except that the spherical cellulose particle having an average particle size of 7 µm was replaced with a spherical cellulose particle (A-2) having an average particle size of 15 µm, and the amount of methyl trimethoxysilane was changed to 40 parts by mass, to obtain a monodisperse, water-repellent powder.

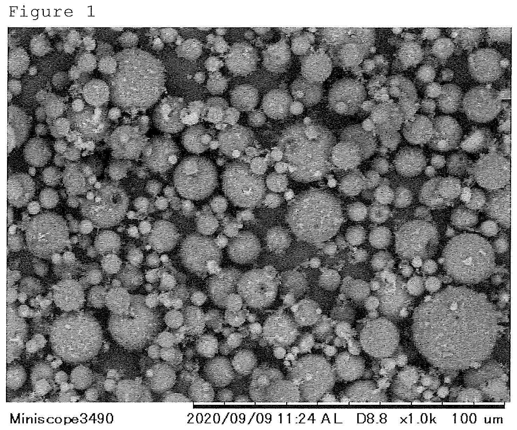

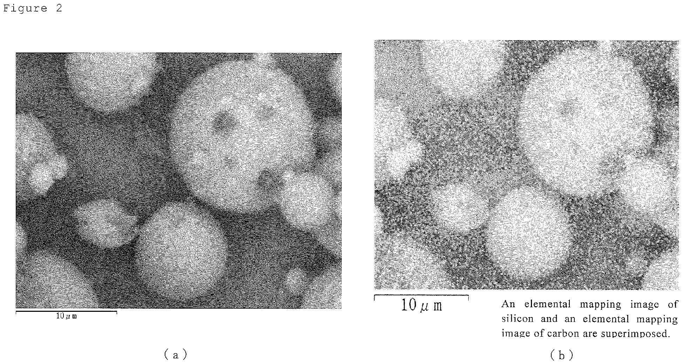

This composite particle was observed using electron microscopy and the presence of a particle having a particle size of about 100 nm was confirmed on the composite particle surface. Further, the infrared absorption spectrum for the resulting composite particle was measured with the potassium bromide disk method using an FT-IR spectrometer, provided by SHIMADZU CORPORATION. As a result, characteristic absorptions were observed at wavenumbers of 2,960 cm⁻¹, 1,260 cm⁻¹, and 1,100 to 1,020 cm⁻¹. This confirmed that the powder obtained included a composite particle in which a polymethyl silsesquioxane was attached to the surface of a granular cellulose particle. FIG. 1 shows an electron microscopy image of the surface of the composite particle. Further, FIG. 2 shows an electron microscopy image (a) of the surface of the composite particle, and a corresponding image (b) in which an elemental mapping image of silicon and an elemental mapping image of carbon, analyzed with a SwiftED3000, provided by Hitachi High-Tech Corporation, are superimposed.

### [Example 5]

The procedures of Example 1 were repeated, except that the spherical cellulose particle having an average particle size of 7 µm was replaced with a spherical cellulose particle (A-2) having an average particle size of 15 µm, and the amount of methyl trimethoxysilane was changed to 15 parts by mass, to obtain a composite particle.

### [Example 6]

The procedures of Example 1 were repeated, except that the cellulose particle in Example 1 was replaced with a spherical cellulose particle (A-3) having an average particle size of 5 µm, to obtain a composite particle.

### [Comparative Examples 1 to 3]

Cellulose particles (A-1), (A-2) and (A-3) were evaluated without being treated with methyl trimethoxysilane.

In Comparative Example 4, a silane resin powder (B-2) with an average particle size of 5 µm, X-52-1621 provided by Shin-Etsu Chemical Co., Ltd., was evaluated.

### [Comparative Example 5]

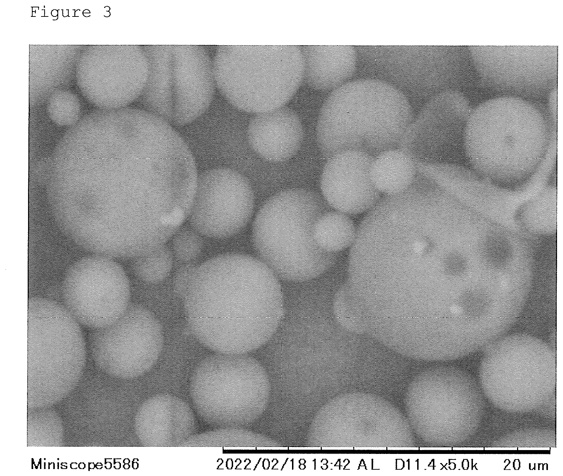

The procedures of Example 1 were repeated, except that the amount of methyl trimethoxysilane was changed to the amount shown in Table 2, to obtain a composite particle. FIG. 3 shows an image of the surface of the composite particle obtained in Comparative Example 5.

The composite particle obtained above was evaluated as follows. The results are shown in Tables 1 and 2.

### <Coverage rate>

The average coverage rate was calculated based on visual observations of electron microscopy images of the surfaces of 10 composite particles. The coverage rate was calculated as follows. Coverage rate = (Covered area / Surface area) x 100

### <Method of evaluating tactile sensation and spread ability>

An appropriate amount of the aforesaid powder sample is applied to a finger, and the finger is placed in contact with a black carbon paper and is slid across the surface in one motion. Tactile sensation was defined as the sensation when the finger was slid across the surface, and spread ability was defined as the length of the resulting trace from sliding the finger, and these were evaluated at the three levels below.
Good: distinguishingly good lubricity was confirmed
Fair: slightly good lubricity was confirmed
Poor: friction against finger was confirmed without lubricity

### <Method of measuring friction coefficient>

About 1 g of the aforesaid powder sample was uniformly spread onto a BIOSKIN plate (provided by Beaulax) to prepare a sample piece having a composite body composed of the composite particle. Using a HEIDON TYPE: 38, provided by Shinto Scientific Co., Ltd., an indenter carrying a 100-g weight was vertically placed in contact with the composite body of the aforesaid sample piece and moved at a speed of 3 cm/min to measure the frictional force. Then, the friction coefficient was calculated from the frictional force. The indenter used had a contact surface with a diameter of 12 mm and was equipped with SUPPLALE, an artificial leather, on the contact surface with the composite.

The static and dynamic friction coefficients of the composite composed of the composite particle are preferably within a range of 0.4 or less, and both the static friction coefficient and the dynamic friction coefficient are within a range of 0.01 to 0.40. For both the static and dynamic friction coefficients, the smaller the value, the better the slide ability. In particular, a value of 0.4 or less provides good smoothness and lubricity, which is preferable.

The static friction coefficient is an indicator of the force required to move an object at rest. A large static friction coefficient is more likely to cause a catching or creaking sensation.

### <Contact angle>

The contact angle was measured as follows.

A sample piece was prepared in the same manner as for the aforesaid friction coefficient measurement. A 10-µm water droplet was dripped onto the sample piece, and the contact angle value after 0.3 seconds was measured using a model CA-D contact angle meter provided by Kyowa Interface Science Co., Ltd. A contact angle of 110° or more is a preferable range for the amount of water repellent.

### <Light scattering intensity, light scattering evaluation>

To 45 g of clear lacquer, was added 5 g of the powder sample and dispersed for 2 minutes at 3,000 rpm with a dispersion mixer. The dispersion was dripped onto a black carbon paper such that a circle with a diameter of 2 cm was formed, and the dispersion was spread with a 5-mil coater to form a coating film. After air drying, the light scattering intensity was measured at three different points on the coating film with a gloss meter, and the average value was used. It can be said that the smaller the reflected light intensity at 85°, the more matte the finish of the powder sample, resulting in better light scattering properties.

**[Table 1]**

| | Example | | | | | |
|---|---|---|---|---|---|---|
| | 1 | 2 | 3 | 4 | 5 | 6 |
| A-1 | 100 | 100 | 100 | | | |
| A-2 | | | | 100 | 100 | |
| A-3 | | | | | | 100 |
| B-1 | 22.5 | 15 | 55 | 40 | 15 | 22.5 |
| B-2 | | | | | | |
| Coverage rate, % | 50 | 40 | 80 | 70 | 60 | 50 |
| Tactile sensation | dry and smooth | dry and smooth | dry and smooth | dry and smoot h | dry and smooth | dry and smooth |
| Static friction coefficient , µs | 0.29 | 0.32 | 0.37 | 0.37 | 0.34 | 0.33 |
| Dynamic friction coefficient , µk | 0.26 | 0.29 | 0.36 | 0.34 | 0.34 | 0.27 |
| Contact angle, ° | 138 | 137 | 135 | 140 | 137 | 128 |
| Spread ability | Good | Good | Good | Good | Good | Good |
| Light scattering intensity, 85° | 2.3 | 2.5 | 3.8 | 12 | 15 | 8.5 |

**[Table 2]**

| | Comparative Example | | | | |
|---|---|---|---|---|---|
| | 1 | 2 | 3 | 4 | 5 |
| A-1 | 100 | | | | 100 |
| A-2 | | 100 | | | |
| A-3 | | | 100 | | |
| B-1 | | | | | 1 |
| B-2 | | | | 100 | |
| Coverage rate, % | 0 | 0 | 0 | 0 | 3 |
| Tactile sensatio n | dry and smoot h | dry and smoot h | dry and smooth | dry and smooth | dry and smooth |
| Static friction coeffici ent, µs | 0.51 | 0.41 | 0.49 | 0.48 | 0.5 |
| Dynamic friction coeffici ent, µk | 0.47 | 0.37 | 0.43 | 0.57 | 0.56 |
| Contact angle, ° | 10 | 10 | 95 | 131 | 10 |
| Spread ability | Fair | Fair | Fair | Fair | Fair |
| Light scatteri ng intensit y, 85° | 4 | 7.5 | 8.3 | 25.4 | 4 |

| | | | | | |
|---|---|---|---|---|---|
| A-1: Cellulose particle with an average particle size of 7 µm A-2: Cellulose particle with an average particle size of 15 µm A-3: Cellulose particle with an average particle size of 5 µm B-1: Methyl trimethoxysilane, KBM13 provided by Shin-Etsu Chemical Co., Ltd. B-2: Silane resin powder with an average particle size of 5 µm, X-52-1621 provided by Shin-Etsu Chemical Co., Ltd. | | | | | |

### [Example 7]

Using the composite particle produced in Example 1, a foundation was prepared according to the blend shown below.

**Foundation**

| Component | | Blending amount (%) |
|---|---|---|
| (1) | Cellulose-siloxane composite particle of Example 1 | 3.0 |
| (2) | Talc treated with acrylic silicone Note 1 | Residual amount |
| (3) | Sericite treated with acrylic silicone | 10.0 |
| (4) | Mica treated with metallic soap | 2.0 |
| (5) | Synthetic phlogopite | 5.0 |
| (6) | Spherical silica powder | 5.0 |
| (7) | Fine titanium oxide particles treated with silicone | 12.5 |
| (8) | Red iron oxide treated with silicone | 0.6 |
| (9) | Yellow iron oxide treated with silicone | 2.0 |
| (10) | Black iron oxide treated with silicone | 0.2 |
| (11) | Titanium oxide treated with silicone | 6.0 |
| (12) | Diisostearyl malate | 2.0 |
| (13) | Glyceryl triisostearate | 0.4 |
| (14) | Methyl polysiloxane | 3.5 |
| (15) | Ultraviolet absorber | 5.0 |
| (16) | Preservative | Appropriate amount |
| (17) | Fragrance | Appropriate amount |

| | | |
|---|---|---|
| Note 1: NS Talc JA-46R-3F provided by Kakuhachi Co., Ltd. | | |

### [Examples 8 to 12 and Comparative Examples 6 to 10]

Foundations were prepared with the same blend as in Example 7, except that the powders were replaced with the composite particles obtained in Examples 2 to 6 and Comparative Examples 1 to 5.

### [Example 13]

A foundation was prepared with the same blend as in Example 7, except that the amount of the composite particle used in Example 7 was changed to 20%.

The evaluations below were performed on the foundations obtained. The results are shown in Tables 3 and 4.

### [Tactile sensation and lubricity]

A usage test was performed by 20 expert panelists to evaluate the cosmetics on evaluation items relating to their tactile sensation and lubricity based on the evaluation point criteria below. Then, the evaluation points given by the panelists were totaled and evaluated based on the evaluation criteria below.

### Evaluation point criteria

5 points: excellent
4 points: good
3 points: fair
2 points: poor
1 point: bad

### Evaluation criteria

Excellent: total score of 80 points or more
Good: total score of 60 points or more, and less than 80 points
Fair: total score of 40 points or more, and less than 60 points
Poor: total score of less than 40 points

**[Table 3]**

| | Example | | | | | | |
|---|---|---|---|---|---|---|---|
| | 7 | 8 | 9 | 10 | 11 | 12 | 13 |
| Types of Composite Particles | Ex. 1 | Ex. 2 | Ex. 3 | Ex. 4 | Ex. 5 | Ex. 6 | Ex. 1 |
| Amount of composite particles in cosmetics | 3.0% | 3.0% | 3.0% | 3.0% | 3.0% | 3.0% | 20% |
| Texture | Excell ent | Excell ent | Excell ent | Excell ent | Excell ent | Excell ent | Excellent |
| Slipperin ess | Excell ent | Excell ent | Excell ent | Excell ent | Excell ent | Excell ent | Excell ent |

**[Table 4]**

| | Comparative Example | | | | |
|---|---|---|---|---|---|
| | 6 | 7 | 8 | 9 | 10 |
| Types of Composite Particles | Com. Ex. 1 | Com. Ex. 2 | Com. Ex. 3 | Com. Ex. 4 | Com. Ex. 5 |
| Amount of composite particles in cosmetics | 3.0% | 3.0% | 3.0% | 3.0% | 3.0% |
| Texture | Good | Good | Good | Good | Good |
| Slipperiness | Fair | Fair | Fair | Fair | Fair |

As shown in Table 4, the foundation using the non-surface-treated cellulose particle or the silane resin powder is poor in terms of tactile sensation and lubricity. Use of the composite particle with an exceedingly small amount of methyl trimethoxysilane relative to the cellulose particle results in a foundation that is poor in terms of tactile sensation and lubricity. Further, use of the composite particle with an exceedingly large amount of methyl trimethoxysilane relative to the cellulose particle has a tendency for the lubricity of the resulting foundation to be poor. In contrast, as shown in Table 3, the cellulose-siloxane composite particle of the present invention provides a foundation that is excellent in terms of tactile sensation and lubricity compared to the non-surface-treated cellulose particle.

When the composite particle of the present invention is blended into and used in a cosmetic, the cosmetic is imparted with slide ability, a soft tactile sensation, water repellency, and the like, and the composite particle of the present invention is suitable to blend into many cosmetics such as hair cosmetics, makeup cosmetics, and sunscreens. Further, the composite powder of the present invention and the cosmetic including the composite powder can be expected to have biodegradable effects.

## Claims

1. A composite particle composed of a particle of cellulose or a cellulose derivative and a polyorganosilsesquioxane attached to the surface of the particle, wherein the polyorganosilsesquioxane is a polymer of 3 to 80 parts by mass of organotrialkoxysilane, relative to 100 parts by mass of the particle.

2. The composite particle according to claim 1, wherein the volume average particle size of the particle of the cellulose or the cellulose derivative is 1 to 300 µm.

3. The composite particle according to claim 1 or 2, wherein the organotrialkoxysilane is a methyl trimethoxysilane.

4. A composite particle composed of a particle of cellulose or a cellulose derivative and a polyorganosilsesquioxane attached to the surface of the particle, wherein 30% or more of the surface area of the particle is covered with the polyorganosilsesquioxane.

5. The composite particle according to claim 4, wherein the volume average particle size of the particle of the cellulose or the cellulose derivative is 1 to 300 µm.

6. The composite particle according to claim 4 or 5, wherein the polyorganosilsesquioxane is a polymer of an organotrialkoxysilane.

7. The composite particle according to claim 6, wherein the organotrialkoxysilane is a methyl trimethoxysilane.

8. A method for preparing a composite particle composed of a particle (A) of cellulose or a cellulose derivative and a polyorganosilsesquioxane attached to the surface of the particle, wherein the method comprising a step of subjecting 3 to 80 parts by mass of an organotrialkoxysilane (B) relative to 100 parts by mass of the particle (A) to a hydrolysis and condensation reaction in the presence of the particle (A), water and an alkali, to form the polyorganosilsesquioxane, and then attach the polyorganosilsesquioxane thus obtained to the surface of the particle (A).

9. The method according to claim 8, further comprising a step of removing water after the step described in claim 8.

10. A cosmetic comprising the composite particle according to any one of claims 1 to 7.

11. The cosmetic according to claim 10, wherein an amount of the composite particle is 1 to 50 % by mass, based on a total amount of the cosmetic.

12. The composite particle according to any one of claims 1 to 7, wherein a static dynamic friction coefficient of a composite body composed of the composite particle is 0.4 or less.
